# EUROPEAN PATENT APPLICATION

(11) **EP 4 464 789 A1**
(43) Date of publication of application: **20.11.2024**
(21) Application number: 22913207.1
(22) Date of filing: 22.06.2022
(51) Int. Cl.: C12Q 1/68, C12P 19/34, C12N 9/99, A61K 31/4035

(54) **METHOD FOR IMPROVING ACTIVITY OF CHEMICALLY MODIFIED TAQ ENZYME AND TAQ ENZYME TREATED USING SAME**

(30) Priority: 30.12.2021 CN 202111658612
(71) Applicant: Daan Gene Co., Ltd., Guangzhou, Guangdong 510665 (CN)
(72) Inventor: JIANG, Xiwen, Guangzhou, Guangdong 510665 (CN); HE, Ling, Guangzhou, Guangdong 510665 (CN); HUANG, Jianfeng, Guangzhou, Guangdong 510665 (CN); NIE, Jianhong, Guangzhou, Guangdong 510665 (CN); PAN, Xufeng, Guangzhou, Guangdong 510665 (CN)
(74) Representative: Diehl & Partner
(86) International application number: PCT/CN2022/100278
(87) International publication number: WO 2023/123918

(57) **Abstract**

A method for improving the activity of a chemically modified Taq enzyme and a Taq enzyme treated using same. The method comprises the steps of performing high performance liquid chromatography on a prepared crude product solution of a chemical modifier and carrying out a modification reaction with a Taq enzyme to be modified. The method for improving the activity of a chemically modified Taq enzyme can greatly improve the activity of a chemically modified Taq enzyme, and in particular the activity of a Taq enzyme modified with the compound represented by formula (I) in a low-cost, simple and convenient way.

## Description

### CROSS-REFERENCE TO RELATED APPLICATION

This patent application claims priority based on a Chinese patent application filed on December 30, 2021, with the application number 202111658612.6, titled *'Method for improving activity of chemically modified taq enzyme and taq enzyme treated using same'.* The aforementioned application is incorporated herein by reference in its entirety.

### TECHNICAL FIELD

The embodiments of the present invention relate to the field of detection, and in particular to a method for improving the activity of chemically modified Taq enzyme and the Taq enzyme treated using the same.

### BACKGROUND

Taq enzyme is the most commonly used tool enzyme for PCR, but because it is still partially active at room temperature, non-specific amplification products often occur, especially when there is a large amount of non-specific DNA in the PCR reaction system. At present, chemical modification methods are often used to modify Taq enzyme. That is, Taq enzyme reversible modifier is used to react with Taq enzyme. The activity of the modified hot start enzyme can be inhibited at room temperature, and its high activity can be quickly restored under high temperature conditions to avoid occurrence of non-specific amplification at room temperature.

In the prior art, dicarboxylic anhydride is often used as a reversible modifier of Taq enzyme to modify Taq enzyme. In addition, patent US2007224611A1 discloses the compound of formula 3 (N-ethoxycarbonyl-2,3-disubstituted butenedimide) as a reversible modifier of thermophilic enzymes, and discloses its preparation and purification methods.

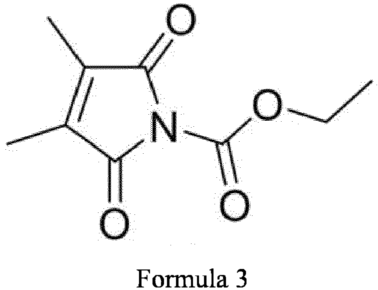

However, the inventor found through research that the Taq enzyme reversible modifier prepared and purified by the above method has poor activity in modifying the Taq enzyme. Therefore, there is still a need in this field to find Taq enzyme reversible modifiers with better modification effects and methods to improve the modification effect of Taq enzyme reversible modifiers.

### SUMMARY OF THE INVENTION

The object of the present invention is to provide a method for improving the activity of chemically modified Taq enzyme.

Another object of the present invention is to provide a Taq enzyme treated by the above method.

Another object of the present invention is to provide a Taq enzyme reversible modifier.

Another object of the present invention is to provide a Taq enzyme modified by the above-mentioned Taq enzyme reversible modifier.

Another object of the present invention is to provide a PCR kit containing the above-mentioned Taq enzyme modified by the Taq enzyme reversible modifier.

The first aspect of the present invention provides a method for improving the activity of chemically modified Taq enzyme, the method comprising the steps of:
passing the prepared crude product solution of the chemical modifier through high performance liquid chromatography, and then performing a modification reaction with the Taq enzyme to be modified.

In some preferred embodiments, the chemical modifier is a compound represented by formula (I)

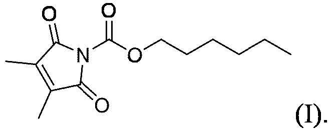

In some preferred embodiments, the method includes the steps of:
(1) Passing the crude product solution of the compound represented by formula (I) through preparative high performance liquid chromatography and collecting the target fraction; and
(2) Extracting and spin evaporating the target fraction obtained in step (1) to obtain the purified compound represented by formula (I); and
(3) Subjecting the purified compound represented by formula (I) obtained in step (2) to a modification reaction with the Taq enzyme to be modified.

In some preferred embodiments, in step (1), the chromatographic column used in the preparative high performance liquid chromatography is a C18 chromatographic column.

In some preferred embodiments, the mobile phase used in the preparative high performance liquid chromatography is a mixed solution of water and methanol.

In some preferred embodiments, the mobile phase used in the preparative high performance liquid chromatography is a mixed solution of water and acetonitrile.

In some preferred embodiments, the preparative high performance liquid chromatography is performed using gradient elution.

In some preferred embodiments, the mobile phase flow rate is 3.5 to 4.5 mL/minute, such as 4 mL/minute.

In some preferred embodiments, the particle size of the C18 chromatography column packing is 2µm to 10µm, such as 2µm, 5µm or 10µm.

In some preferred embodiments, the procedure of the gradient elution is:
within the time period t, the volume percentage of acetonitrile in the mobile phase is changed at a constant speed from 10% to 100%; the t is 25 to 35 minutes, such as 30 minutes.

In some preferred embodiments, the target fraction is an eluate at a retention time of 15 to 19 minutes, for example, an eluate at a retention time of 17 to 18 minutes.

In some preferred embodiments, in step (2), the extraction agent used in the extraction is dichloromethane.

In some preferred embodiments, in step (3), the modification reaction includes the step of mixing the purified compound represented by formula (I) obtained in step (2) with the Taq enzyme to be modified.

In some preferred embodiments, the mixing is performed using an oscillator, and the oscillation time of the oscillator is 15 to 30 seconds, such as 20 seconds.

A second aspect of the present invention provides a Taq enzyme treated by the method described in the first aspect of the present invention.

Compared with the prior art, the present invention at least has the following beneficial effects:
The method for improving the activity of chemically modified Taq enzyme provided by the first aspect of the present invention can greatly improve the activity of chemically modified Taq enzyme, especially the activity of Taq polymerase modified with the compound represented by formula (I) in a low-cost, simple and convenient way.

It is to be understood that within the scope of the present invention, the various technical features of the present invention and the various technical features specifically described hereinafter (as in the embodiments) may be combined with each other to form a new or preferred technical solution. Due to space limitations, we will not repeat them here.

### DESCRIPTION OF DRAWINGS

One or more embodiments are exemplified by the pictures in the corresponding drawings, and these exemplary illustrations do not constitute limitations to the embodiments.
Figure 1 is an HPLC diagram of pure hexyl 3,4-dimethyl-2,5-dioxo-2,5-dihydro-1H-pyrrole-1-carboxylate in an embodiment of the present invention;
Figure 2 is an LC-MS spectrum of pure hexyl 3,4-dimethyl-2,5-dioxo-2,5-dihydro-1H-pyrrole-1-carboxylate in an embodiment of the present invention;
Figure 3 is a ¹H MNR spectrum of hexyl 3,4-dimethyl-2,5-dioxo-2,5-dihydro-1H-pyrrole-1-carboxylate in an embodiment of the present invention;
Figure 4 is an HPLC diagram of pure hexyl 3,4-dimethyl-2,5-dioxo-2,5-dihydro-1H-pyrrole-1-carboxylate in an embodiment of the present invention;
Figure 5 is an HPLC diagram of pure hexyl 3,4-dimethyl-2,5-dioxo-2,5-dihydro-1H-pyrrole-1-carboxylate in an embodiment of the present invention;
Figure 6 is a melting curve diagram of enzyme activity of hexyl 3,4-dimethyl-2,5-dicarbonyl-2,5-dihydro-1H-pyrrole-1 -carboxylate modified DNA polymerase, wild-type DNA polymerase and no DNA polymerase in an embodiment of the present invention.

### DETAILED DESCRIPTION OF THE INVENTION

After detailed research, the inventor found that the activity of Taq enzyme modified by butenedimide modifiers (formula A below) is significantly better than that of commonly used dicarboxylic anhydride-modified Taq enzyme. Among butenedimide modifiers, the N-terminal substituent R¹ significantly affects the modification effect. For example, the activity of Taq enzyme modified with hexyl 3,4-dimethyl-2,5-dicarbonyl-2,5-dihydro-1H-pyrrole-1-carboxylate (3,4-dimethyl-2,5-dicarbonyl-2,5-dihydro-1H-pyrrole-1 -carboxylic acid hexyl ester) verified in the test examples of the present invention is significantly better than Taq enzyme modified with N-ethoxycarbonyl-2,3-disubstituted butenedimide. ( 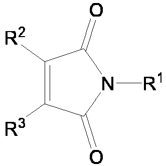 Formula A, wherein R¹, R² and R³ are independently selected from alkyl, alkenyl, alkynyl, cycloalkyl, hydroxyl, alkoxy, halogen, amine, amino, nitro, amide or acyloxy.)

The activity of Taq enzyme modified by hexyl 3,4-dimethyl-2,5-dicarbonyl-2,5-dihydro-1H-pyrrole-1-carboxylate was significantly better than that of dicarboxylic anhydride or N-ethoxycarbonyl- 2,3-disubstituted butenedimide modified Taq enzyme.

In addition, the inventor also found that the butenedimide modifier treated by the method of improving the activity of chemically modified Taq enzyme according to the present invention has better enzyme activity and high stability. Preferably, the Taq polymerase modified with hexyl 3,4-dimethyl-2,5-dicarbonyl-2,5-dihydro-1H-pyrrole-1-carboxylate, processed by the method described in this invention for improving the activity of chemically modified Taq polymerase, shows significantly higher activity than the unprocessed Taq polymerase modified with hexyl 3,4-dimethyl-2,5-dicarbonyl-2,5-dihydro-1H-pyrrole-1-carboxylate.

Some embodiments of the present invention provide a Taq enzyme reversible modifier, and the structure of the Taq enzyme reversible modifier is shown in formula (a)

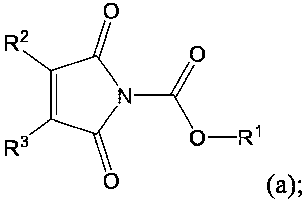

Wherein, R¹ is C₃₋₆ alkyl;
R² and R³ are each independently C₁₋₆ alkyl.
In some preferred embodiments,
R¹ is unsubstituted C₃₋₆ linear alkyl;
And/or, R² and R³ are each independently C₁₋₄ alkyl.

In some preferred embodiments,
R¹ is n-propyl, n-butyl, n-pentyl or n-hexyl.

In some preferred embodiments,
R² and R³ are independently methyl, ethyl, n-propyl, isopropyl, n-butyl, sec-butyl, isobutyl and tert-butyl.

In some preferred embodiments, R¹ is n-hexyl.

In some preferred embodiments, the structure of the Taq enzyme reversible modifier is shown in formula (I)

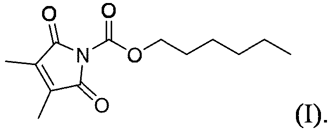

In some preferred embodiments, the Taq enzyme reversible modifier is obtained by purifying the crude product of hexyl 3,4-dimethyl-2,5-dicarbonyl-2,5-dihydro-1H-pyrrole-1-carboxylate using high performance liquid chromatography.

Some embodiments of the present invention provide a chemically modified Taq enzyme, which is modified by the Taq enzyme reversible modifier described in the first aspect of the present invention.

Some embodiments of the present invention provide a PCR kit, which is characterized in that the PCR kit includes the chemically modified Taq enzyme described in the second aspect of the present invention.

Some embodiments of the present invention provide a method for preparing the chemically modified Taq enzyme provided in the third aspect of the present invention. The method includes the steps of: mixing wild-type Taq enzyme and the Taq enzyme reversible modifier described in the first aspect of the present invention.

In some preferred embodiments, the mixing is performed in an oscillator, and the mixing lasts for 15 to 30 seconds.

Some embodiments of the present invention provide a method for improving the activity of chemically modified Taq enzyme, which method includes the steps of:
Passing the prepared crude product solution of the chemical modifier is passed through high performance liquid chromatography, and then being subjected to a modification reaction with the Taq enzyme to be modified.

In some preferred embodiments, the chemical modifier is a compound represented by formula (I)

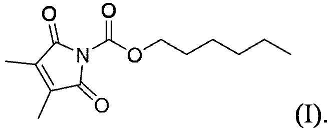

In some preferred embodiments, the method includes the steps of:
(1) Passing the crude product solution of the compound represented by formula (I) through preparative high performance liquid chromatography, and collecting the target fraction; and
(2) Extracting and spin evaporating the target fraction obtained in step (1) to obtain the purified compound represented by formula (I); and
(3) Subjecting the purified compound represented by formula (I) obtained in step (2) to a modification reaction with the Taq enzyme to be modified.

In some preferred embodiments, in step (1), the chromatographic column used in the preparative high performance liquid chromatography is a C18 chromatographic column.

In some preferred embodiments, the mobile phase used in the preparative high performance liquid chromatography is a mixed solution of water and acetonitrile.

In some preferred embodiments, the preparative high performance liquid chromatography is performed using gradient elution.

In some preferred embodiments, the mobile phase flow rate is 3.5 to 4.5 mL/minute, such as 4 mL/minute.

In some preferred embodiments, the particle size of the C18 chromatography column packing is 2 µm to 10 µm, such as 2 µm, 5 µm or 10 µm.

In some preferred embodiments, the procedure of the gradient elution is: within the time period t, the volume percentage of acetonitrile in the mobile phase is changed at a constant speed from 10% to 100%; the t is 25 to 35 minutes, such as 30 minutes.

In some preferred embodiments, the target fraction is an eluate at a retention time of 15 to 19 minutes, for example, an eluate at a retention time of 17 to 18 minutes.

In some preferred embodiments, in step (2), the extraction agent used in the extraction is dichloromethane.

In some preferred embodiments, in step (3), the modification reaction includes the step of mixing the purified compound represented by formula (I) obtained in step (2) with the Taq enzyme to be modified.

In some preferred embodiments, the mixing is performed using an oscillator, and the oscillation time of the oscillator is 15 to 30 seconds, such as 20 seconds.

In order to make the purpose, technical solutions and advantages of the embodiments of the present invention clearer, the present invention will be further described below in conjunction with specific embodiments. It should be understood that these examples are only used to illustrate the invention and are not intended to limit the scope of the invention. Experimental methods without specifying specific conditions in the following examples usually follow conventional conditions or conditions recommended by the manufacturer. Unless otherwise stated, percentages and parts are by weight. The experimental materials and reagents used in the following examples can be obtained from commercial sources unless otherwise specified.

Unless otherwise specified, technical and scientific terms used herein have the same meanings commonly understood by one of ordinary skill in the art to which this application belongs. It should be noted that the terms used herein are only for describing specific embodiments and are not intended to limit the exemplary embodiments of the present application.

### Example 1. Preparation of hexyl 3,4-dimethyl-2,5-dicarbonyl-2,5-dihydro-1H-pyrrole-1-carboxylate

To a solution of 30.5g 2,3-dimethylmaleic anhydride in 1L N, N-dimethylformamide at room temperature, 77.6g 1,1,1,3,3,3-hexamethyl disilazane was added. The mixture was heated to 100°C and stirred for 30 min. Then it was cooled to room temperature, 3 L of water was added to quench the reaction, and the resulting solution was extracted with 10 L of ethyl acetate. The combined organic extracts were washed with 3 L of brine, dried over Na₂SO₄, filtered, and concentrated in vacuo to give a crude residue. The residue was purified by silica gel column chromatography (eluent: hexane/AcOEt=3/1) to obtain 23.3 g of crude 2,3-dimethylmaleimide as a white solid, which was set aside for use. At 0°C, 34.8 g of triethylamine and 42.5g of n-hexyl chloroformate was added to a solution of 23.3g of crude 2,3-dimethylmaleimide in 170ml of anhydrous dichloromethane. After completion, the temperature was raised to room temperature, and the mixture was stirred for 20 minutes. The reaction was quenched by adding 3 L of saturated aqueous NH₄Cl solution and the resulting solution was extracted with 10 L of ethyl acetate. The combined organic extracts were washed with 3L of brine, dried over Na₂SO₄, filtered, and concentrated in vacuo to obtain the crude product of hexyl 3,4-dimethyl-2,5-dicarbonyl-2,5-dihydro-1H-pyrrole-1 -carboxylate.

### Example 2. Refined of hexyl 3,4-dimethyl-2,5-dicarbonyl-2,5-dihydro-1H-pyrrole-1-carboxylate

### Purification way 1:

The crude hexyl 3,4-dimethyl-2,5-dioxo-2,5-dihydro-1H-pyrrole-1-carboxylate was dissolved in an acetonitrile-water solution, filtered through a 0.45µm microporous membrane, C18 was used as the stationary phase with a packing particle size of 2µm, the system was equilibrated for 5 minutes with a mobile phase of water : acetonitrile = 90: 10, the sample was loaded, the target component was collected after about 7 minutes of elution, collected for about 1 minute, the collected fraction was extracted with dichloromethane, the organic phase was collected, and after processing with a rotary evaporator, a colorless transparent liquid was obtained which is the pure hexyl 3,4-dimethyl-2,5-dioxo-2,5-dihydro-1H-pyrrole-1-carboxylate. Purity is 99.4% as determined by HPLC.

HPLC analysis and detection methods are as follows:

| | |
|---|---|
| Instrument | Waters e2695 analytical liquid phase |
| Column | SunFire C18 100Å, 5 µm, 10 mm x 250 mm |
| Injection volume | 100µL |
| Flow rate | 4mL/min |
| Running time | 30min |
| Detection wavelength | 254nm |
| Mobile phase | Acetonitrile, Water |
| Gradient | The volume percentage of acetonitrile changes from 10% to 100% at a constant gradient from 0 to 30 minutes. |

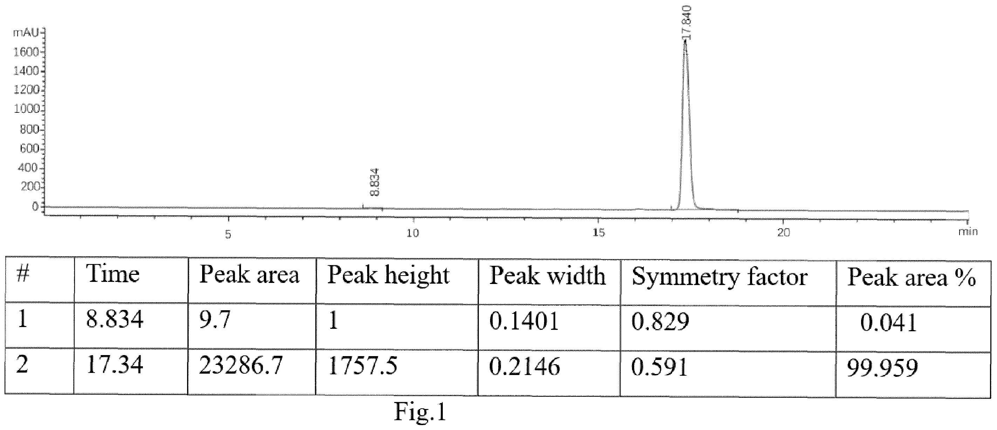

The chromatogram obtained by the above HPLC is shown in Figure 1.

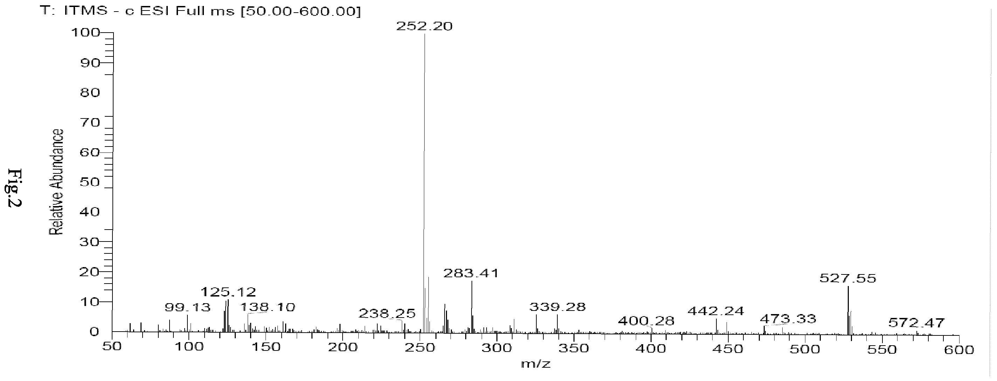

The LC-MS spectrum of purified hexyl 3,4-dimethyl-2,5-dioxo-2,5-dihydro-1H-pyrrole-1-carboxylate is shown in Figure 2

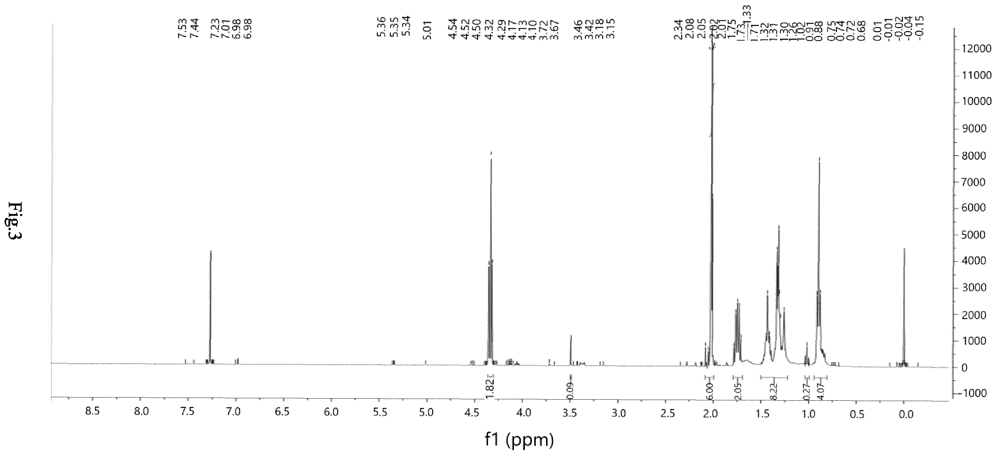

The ¹H MNR spectrum of purified hexyl 3,4-dimethyl-2,5-dioxo-2,5-dihydro-1H-pyrrole-1-carboxylate is shown in Figure 3.

### Purification way 2:

The crude hexyl 3,4-dimethyl-2,5-dioxo-2,5-dihydro-1H-pyrrole-1-carboxylate from Example 1 was dissolved in an acetonitrile/water solution, filtered through a 0.45µm microporous membrane, C18 was used as the stationary phase with a packing particle size of 5µm, the system was equilibrated for 15 minutes with a mobile phase of water : acetonitrile = 90:10, the sample was load, the target component was collected after about 15 minutes of elution, collected for about 2 minutes, the collected fraction was extracted with dichloromethane, the organic phase was collected, and after processing with a rotary evaporator, a colorless transparent liquid was obtained which was the pure hexyl 3,4-dimethyl-2,5-dioxo-2,5-dihydro-1H-pyrrole-1-carboxylate. Upon testing, the purity is found to be 99.2%.

HPLC analysis and detection methods are as follows:

| | |
|---|---|
| Instrument | Waters e2695 analytical liquid phase |
| Column | SunFire C18 100Å, 5 µm, 10 mm x 250 mm |
| Injection volume | 100µL |
| Flow rate | 4mL/min |
| Running time | 30min |
| Detection wavelength | 254nm |
| Mobile phase | Acetonitrile, Water |
| Gradient | The volume percentage of acetonitrile changes from 10% to 100% at a constant gradient from 0 to 30 minutes. |

### Purification way 3:

The crude hexyl 3,4-dimethyl-2,5-dioxo-2,5-dihydro-1H-pyrrole-1-carboxylate was dissolved in a 20% acetonitrile/water solution, filtered through a 0.45µm microporous membrane, C18 was used as the stationary phase with a packing particle size of 10µm, the system was equilibrated for 20 minutes with a mobile phase of water: acetonitrile = 90: 10, the sample was loaded, the target component was collected after about 35 minutes of elution, collected for about 5 minutes, the collected fraction was extracted with dichloromethane, the organic phase was collected, and after processing with a rotary evaporator, a colorless transparent liquid was obtained which was the pure hexyl 3,4-dimethyl-2,5-dioxo-2,5-dihydro-1H-pyrrole-1-carboxylate. Upon testing, the purity is found to be 99.1%.

HPLC analysis and detection methods are as follows:

| | |
|---|---|
| Instrument | Waters e2695 analytical liquid phase |
| Column | SunFire C18 100Å, 5 µm, 10 mm x 250 mm |
| Injection volume | 100µL |
| Flow rate | 4mL/min |
| Running time | 30min |
| Detection wavelength | 254nm |
| Mobile phase | Acetonitrile, Water |
| Gradient | The volume percentage of acetonitrile changes from 10% to 100% at a constant gradient from 0 to 30 minutes. |

### Purification way 4:

In the refining method 4, the method of processing crude hexyl 3,4-dimethyl-2,5-dioxo-2,5-dihydro-1H-pyrrole-1-carboxylate was substantially the same as the Purification way 3, except that, in the HPLC analysis and detection step, the mobile phases used in gradient elution were different. The mobile phase used in the refining method 4 was methanol and water. In the initial mobile phase, the volume ratio of methanol and water is 10:90. The volume percentage of methanol in the final mobile phase changed from 10% to 100% at a constant gradient from 0 to 30 minutes.

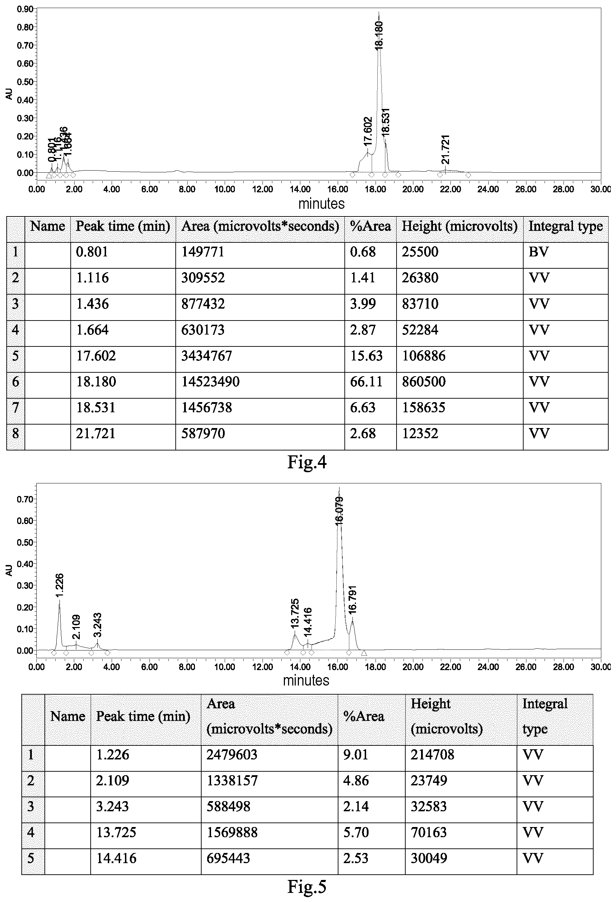

The obtained HPLC spectrum is shown in Figure 4.

### Refining method 5:

In the refining method 5, the method of processing crude hexyl 3,4-dimethyl-2,5-dioxo-2,5-dihydro-1H-pyrrole-1-carboxylate was substantially the same as the refining method 4, except that, in the HPLC analysis and detection step, the volume ratio of methanol and water in the initial mobile phase was 50:50, and the volume percentage of methanol changed from 50% to 100% at a constant gradient from 0 to 30 minutes.

The obtained HPLC spectrum is shown in Figure 5

### Example 3. Modification of Taq enzyme with crude and refined hexyl 3,4-dimethyl-2,5-dicarbonyl-2,5-dihydro-1H-pyrrole-1-carboxylate

The crude product of hexyl 3,4-dimethyl-2,5-dicarbonyl-2,5-dihydro-1H-pyrrole-1-carboxylate was taken and mixed with wild-type DNA polymerase, and shook on an oscillator for 20 seconds. A DNA polymerase modified by a crude product of hexyl 3,4-dimethyl-2,5-dicarbonyl-2,5-dihydro-1H-pyrrole-1-carboxylate was obtained.

By replacing the crude product of hexyl 3,4-dimethyl-2,5-dicarbonyl-2,5-dihydro-1H-pyrrole-1-carboxylate with the pure hexyl 3,4-dimethyl-2,5-dicarbonyl-2,5-dihydro-1H-pyrrole-1-carboxylate obtained in the Purification ways 1, 2, and 3 in Example 2, a DNA polymerase modified with the pure hexyl 3,4-dimethyl-2,5-dicarbonyl-2,5-dihydro-1H-pyrrole-1 -carboxylate was obtained.

### Example 4. PCR reaction system preparation and PCR experimental method

Human single-stranded nucleic acid fragments were used as templates, the DNA polymerase activity of the Taq DNA polymerase to be tested was tested. The specific steps were as follows:
(1) Design and synthesis of primers and templates

Referring to the chromosome 16 gene sequence registered in GenBank, a single-stranded nucleic acid sequence and a primer complementary to the single-stranded nucleic acid were designed:

Single-stranded nucleic acid sequence:

GAGCCCGCCGCCCGGCCCCGCGCAGGCCCCGCCCGGGACTCCC
CTGCGGTCCAGGCCGCGCCCCGGGCTCCGCGCCAGCCAATGAGCGC
CGCCCGGCCGGGCGTGCCCCCGCGCCCCAAGCATAAACCCTGGCGC
GCTCGCGGGCCGGCACTCTTCTGGTCCCCACAGACTC (SEQ ID NO:
1)

Primer sequence: 5'-GAGTCTGTGGGGAG-3' (SEQ ID NO: 2), both were artificially synthesized.

### (2) Template and primer processing before PCR reaction

The synthesized templates and primers were diluted appropriately to 50 pmol/ul with sterilized purified water or 1 *TE (pH8.0), and stored for later use.

The optimal concentration of magnesium ions was 5mmol/L, the optimal dosage of hot-start DNA polymerase (or hot-start DNA polymerase modification) was 5U/reaction, the optimal dosage of UNG enzyme was 0.1U/reaction, and the optimal concentration of deoxyribonucleoside triphosphates (dNTPs) was 0.24mmol/L.

### (3) Preparation of PCR reaction system

A PCR reaction tube was taken and 5µL of 10x PCR buffer was added, magnesium chloride was added to make the working solution concentration of 5mmol/L, dNTPs were added to make the working solution concentration of 0.24mmol/L, fluorescent dye was added to make the working solution concentration of 1%, template was added to make the working solution concentration of 50 pmol/ul, primers were added to make the working solution concentration of 5pmol/ul, the DNA polymerase to be tested (or DNA polymerase modified with hexyl 3,4-dimethyl-2,5-dicarbonyl-2,5-dihydro-1H-pyrrole-1-carboxylate) was added to make the working solution concentration of 2.5U, and then purified water was added to bring the total volume up to 50µL.

### (4) PCR reaction

Each system prepared in the above step (3) was put into a fluorescence quantitative PCR instrument for reaction. The reaction condition was: incubation at 37°C for 60 minutes.

### Test example 1. Blocking activity test of DNA polymerase

The refined DNA polymerase modified with hexyl 3,4-dimethyl-2,5-dicarbonyl-2,5-dihydro-1H-pyrrole-1-carboxylate was used, and its blocking activity on DNA polymerase was measured.

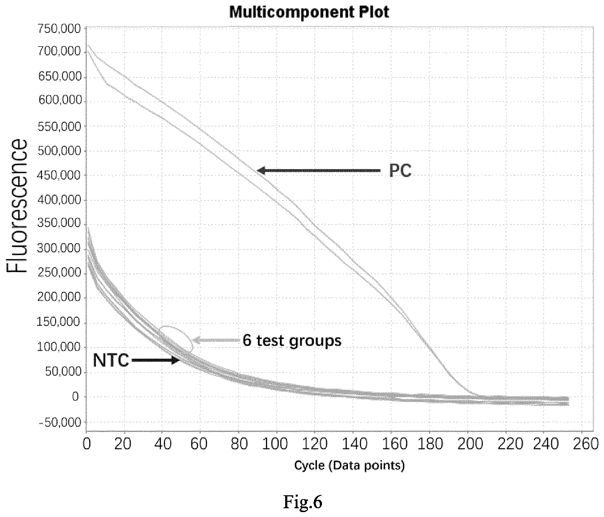

Partially complementary primers were added to the qPCR system, and then the DNA polymerase modified with the refined hexyl 3,4-dimethyl-2,5-dicarbonyl-2,5-dihydro-1H-pyrrole-1-carboxylate was added thereto, with unmodified bare DNA polymerase as a positive control and a complete absence of enzyme as a negative control. It was incubated at 37°C for 60 minutes to obtain the melting curve for the bare enzyme activity test, as shown in Figure 6.

In Figure 6, NTC represents the system without enzyme, and PC represents the bare enzyme system. As can be seen from Figure 1, the curve of the enzyme blocked by hexyl 3,4-dimethyl-2,5-dicarbonyl-2,5-dihydro-1H-pyrrole-1-carboxylate essentially coincides with the no-enzyme curve, indicating that hexyl 3,4-dimethyl-2,5-dicarbonyl-2,5-dihydro-1H-pyrrole-1-carboxylate can completely block the activity of the polymerase.

### Test example 2, enzyme activity test

The crude hexyl 3,4-dimethyl-2,5-dicarbonyl-2,5-dihydro-1H-pyrrole-1-carboxylate, and the refined hexyl 3,4-dimethyl-2,5-dicarbonyl-2,5-dihydro-1H-pyrrole-1-carboxylate obtained in the refining methods 1-5 were used to modify four hot-start enzymes (ABI, Bioline, Biog, and domestic hot-start Taq enzyme), with each chemical modifier being added in amounts of 0.8x, 1.0x, and 1.2x, respectively.

Partially complementary primers were added into the qPCR system, and then to the system the hot-start Taq enzymes (ABI, Bioline, Biog, and domestic hot-start Taq enzyme) modified with the crude hexyl 3,4-dimethyl-2,5-dicarbonyl-2,5-dihydro-1H-pyrrole-1-carboxylate, and the hot-start Taq enzymes modified with the refined hexyl 3,4-dimethyl-2,5-dicarbonyl-2,5-dihydro-1H-pyrrole-1-carboxylate in the refining methods 1-5 as the experimental group were added. The hot-start Taq enzymes modified with the crude hexyl 3,4-dimethyl-2,5-dicarbonyl-2,5-dihydro-1H-pyrrole-1-carboxylate was used as the control group. It was incubated at 37 degrees for 60 minutes. The configuration of the modified hot-start DNA polymerase is as shown in Table 1.

**Table 1. Preparation of DNA polymerase modifications**

| Group | Inhibitor stock solution concentration | Added volume of inhibitor stock solution | Modification Buffer volume |
|---|---|---|---|
| Control group | 105.7mg/ml | 60ul | 1.5ml |
| Experimental group crude (0.8x) | | 48ul | |
| Experimental group refining method 1(0.8x) | | 48ul | |
| Experimental group refining method 2(0.8x) | | 48ul | |
| Experimental group refining method 3(0.8x) | | 48ul | |
| Experimental group refining method 4(0.8x) | | 48ul | |
| Experimental group refining method 5(0.8x) | | 48ul | |
| Experimental group crude(1.0x) | | 60ul | |
| Experimental group refining method 1(1.0x) | | 60ul | |
| Experimental group refining method 2(1.0x) | | 60ul | |
| Experimental group refining method 3(1.0x) | | 60ul | |
| Experimental group refining method 4(1.0x) | | 60ul | |
| Experimental group refining method 5(1.0x) | | 60ul | |
| Experimental group crude(1.2x) | | 72ul | |
| Experimental group refining method 1(1.2x) | | 72ul | |
| Experimental group refining method 2(1.2x) | | 72ul | |
| Experimental group refining method 3(1.2x) | | 72ul | |
| Experimental group refining method 4(1.2x) | | 72ul | |
| Experimental group refining method 5(1.2x) | | 72ul | |

The test results of the experimental group and the control group are shown in Table 2 below.

**Table 2**

| Group | ABI Taq enzyme | Bioline Taq enzyme | Biog Taq enzyme | Domestic Taq enzyme |
|---|---|---|---|---|
| Experimental group crude (0.8x) | 14.22 | 17.77 | 21.06 | 24.51 |
| Experimental group refining method 1(0.8x) | 13.61 | 16.99 | 20.28 | 23.55 |
| Experimental group refining method 2(0.8x) | 13.54 | 16.86 | 20.19 | 23.42 |
| Experimental group refining method 3(0.8x) | 13.50 | 16.82 | 20.15 | 23.37 |
| Experimental group refining method 4(0.8x) | 13.88 | 17.06 | 20.47 | 24.01 |
| Experimental group refining method 5(0.8x) | 13.94 | 17.45 | 20.86 | 24.32 |
| Experimental group crude(1.0x) | 14.05 | 17.51 | 20.86 | 24.30 |
| Experimental group refining method 1(1.0x) | 13.18 | 16.59 | 19.97 | 23.41 |
| Experimental group refining method 2(1.0x) | 13.12 | 16.56 | 19.94 | 23.33 |
| Experimental group refining method 3(1.0x) | 13.22 | 16.56 | 20.01 | 23.29 |
| Experimental group refining method 4(1.0x) | 13.68 | 16.94 | 20.14 | 24.01 |
| Experimental group refining method 5(1.0x) | 13.44 | 17.05 | 20.43 | 24.12 |

Those skilled in the art will understand that the above embodiments are specific examples of implementing the present invention, and that in practical applications, various changes can be made in form and detail without departing from the spirit and scope of the invention.

## Claims

1. A method for improving the activity of chemically modified Taq enzyme, wherein the method includes the steps of:
passing the prepared crude product solution of the chemical modifier through high performance liquid chromatography, and then being subjected to a modification reaction with the Taq enzyme to be modified.

2. The method according to claim 1, wherein the chemical modifier is a compound represented by formula (I)

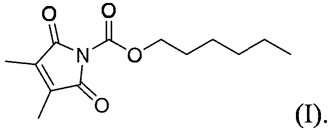

3. The method according to claim 2, wherein the method includes the steps of:
(1) passing the crude product solution of the compound represented by formula (I) through preparative high performance liquid chromatography, and collecting a target fraction; and
(2) extracting and spin evaporating the target fraction obtained in step (1) to obtain the purified compound represented by formula (I); and
(3) subjecting the purified compound represented by formula (I) obtained in step (2) to a modification reaction with the Taq enzyme to be modified.

4. The method according to claim 3, wherein in step (1), the chromatographic column used in the preparative high performance liquid chromatography is a C18 chromatographic column;
and/or, the mobile phase used in the preparative high performance liquid chromatography is a mixed solution of water and acetonitrile;
and/or, the preparative high performance liquid chromatography is performed using a gradient elution method;
and/or, the mobile phase flow rate is 3.5 to 4.5 mL/minute, such as 4 mL/minute.

5. The method according to claim 4, wherein the C18 chromatography column packing particle size is 2 µm ~ 10 µm, such as 2 µm, 5 µm or 10 µm.

6. The method according to claim 4, wherein the procedure of the gradient elution is: within the time period t, the volume percentage of acetonitrile in the mobile phase is changed from 10% to 100% at a uniform speed;
t is 25 to 35 minutes, for example 30 minutes.

7. The method of claim 6, wherein the target fraction is an eluate at a retention time of 15 to 19 minutes, for example, an eluate at a retention time of 17 to 18 minutes.

8. The method according to claim 3, wherein in step (2), the extraction agent used in the extraction is dichloromethane.

9. The method of claim 3, wherein in step (3), the modification reaction includes a step of mixing the purified compound of formula (I) obtained in step (2) with the Taq enzyme to be modified.

10. The method according to claim 9, wherein the mixing is performed using an oscillator, and the oscillation time of the oscillator is 15 to 30 seconds, such as 20 seconds.

11. A Taq enzyme treated by the method of any one of claims 1 to 10.
